# FASCICULE DE BREVET EUROPEEN

(11) **EP 3 893 833 B1**
(45) Date de publication et mention de la délivrance du brevet: **09.07.2025**
(21) Numéro de dépôt: 19816777.7
(22) Date de dépôt: 12.12.2019
(51) Int. Cl.: A61K 8/34, A61Q 13/00, A61K 8/81, A61K 8/92, A61K 8/04

(54) **GELÉE DE PARFUM**
PARFÜMGEL
PERFUME GEL

(30) Priorité: 12.12.2018 FR 1872748
(43) Date de publication de la demande: 20.10.2021
(73) Titulaire: L'OREAL, 75008 Paris (FR)
(72) Inventeur: PERRAULT, Véronique, 94152 CHEVILLY LA RUE (FR); NOEL, Christine, 94152 CHEVILLY LA RUE (FR)
(74) Mandataire: Lavoix
(86) Numéro de dépôt international: PCT/EP2019/084963
(87) Numéro de publication internationale: WO 2020/120704

(56) Documents cités:
- WO-A1-2004/098556
- FR-A1- 2 922 764
- FR-A1- 2 967 906
- US-B1- 8 921 303
- LUBRIZOL: "TECHNICAL DATA SHEET-237", 16 September 2009 (2009-09-16), XP055598465, Retrieved from the Internet <URL:https://www.lubrizol.com/-/media/Lubrizol/Life-Sciences/Documents/TDS/Neutralizing-Carbopol-and-Pemulen-in-Aqueous-and-Hydroalcoholic-Systems.pdf> [retrieved on 20190621]
- LUBRIZOL: "TECHNICAL DATA SHEET Technical Data Sheet Carbopol Ultrez 21 Polymer Typical Properties", 6 November 2002 (2002-11-06), XP055598467, Retrieved from the Internet <URL:https://www.ge-iic.com/files/fichas%20productos/Carbopol_Ultrez_21_hoja_tecnica.pdf> [retrieved on 20190621]

## Description

La présente invention concerne une composition parfumante sous forme de gel hydroalcoolique comprenant au moins 5% en poids par rapport au poids total de composition d'au moins une substance parfumante.

Les compositions parfumantes permettent de parfumer le corps par application par touche. Parmi ces compositions, on trouve notamment des formules sous forme de gel hydroalcoolique.

Les formules sous forme de gel hydroalcoolique actuellement disponibles sur le marché comprennent généralement une forte quantité d'alcool (i.e. au moins 30% en poids), pour assurer leur conservation sur le long terme.

Cependant, de telles formules contiennent en général une faible quantité de parfum (par exemple de l'ordre de 1 % en poids), qui s'estompe donc rapidement avec le temps.

US8921303 B1 divulgue des compositions parfumantes comportant de l'alcool, 5% d'une huile essentielle, et 1.5% du copolymère hydroxyethyl acrylate/sodium acryloyldimethyltaurate. FR2922764 A1 divulgue des compositions parfumantes comprenant 5.8% d'un parfum; 75.5% éthanol, et un copolymère de monomère (A) acide acrylamido 2-méthylpropane sulfonique partiellement ou totalement neutralisé et d'un monomère (B) qui est hydrophobe, donc non ionique.

Il existe ainsi un besoin pour des compositions parfumantes à base de gel hydroalcoolique qui contiennent une quantité importante de parfum et qui soient stables et homogènes.

L'invention a pour but de résoudre les problèmes techniques précités. En particulier, un objectif consiste en la fourniture d'une composition cosmétique parfumante sous forme de gel hydroalcoolique, qui soit stable et homogène. Une telle composition parfumante contient une très forte concentration de parfum, et permet le parfumage par touche sur les points de pulsation (i.e. zones de la peau situées derrière les oreilles, à l'intérieur des poignets, à l'intérieur du coude), est agréable à appliquer (application crémeuse), et présente un fini poudré après l'application sur la peau (pas rêche, pas collant, doux et glissant). La composition parfumante présente également une fraîcheur à l'application.

Les inventeurs ont maintenant découvert, de manière surprenante, que l'association de deux polymères gélifiants spécifiques, dans un milieu aqueux comprenant de l'éthanol et contenant une très forte concentration de parfum, permet d'obtenir des gels hydroalcooliques stables (i.e. après un mois à la température 45°C) et agréables à l'application, présentant un toucher frais et un fini poudré. De manière surprenante, la composition reste homogène (pas de déphasage observé).

La présente invention a donc pour objet une composition parfumante, notamment cosmétique et/ou dermatologique, comprenant:
un milieu aqueux physiologiquement acceptable comprenant au moins de l'éthanol ;
   i) au moins un polymère sulfonique choisi parmi les homopolymères d'acide acrylamido-2-méthyl propane sulfonique ou ses sels et les copolymères d'acide acrylamido-2-méthyl propane sulfonique ou ses sels et d'un ou plusieurs monomères non ioniques ;
   ii) au moins un copolymère réticulé d'acide acrylique et d'acrylate d'alkyle en C10-C30 ; et
   iii) éventuellement au moins un polymère épaississant organique différent de i) et ii) ; et
au moins 5% en poids par rapport au poids total de composition d'au moins une substance parfumante.

La présente invention se rapporte également à une méthode de soin cosmétique et/ou esthétique, notamment pour parfumer, des matières kératiniques comprenant l'application topique sur les matières kératiniques, de préférence sur la peau, d'une composition selon l'invention.

Par « *matières kératiniques* », on entend la peau et/ou les lèvres et/ou les cheveux.

Par « *au moins un* » on entend un ou plusieurs. La composition selon l'invention est sous forme de gel hydroalcoolique.

Par « *gel hydroalcoolique* », on entend un gel comprenant un milieu aqueux comprenant au moins de l'éthanol. La composition selon l'invention est notamment sous forme de gel, et comprend des « polymères gélifiants » spécifiques. Par « polymère gélifiant », on entend au sens de la présente invention un polymère qui permet d'augmenter significativement la viscosité de la composition.

### Viscosité

Les compositions selon l'invention présentent de préférence une viscosité comprise entre 9 et 40 Poises (0,9 à 4 *Pa·s*)*,* de préférence entre 26 et 35 Poises Poises (2,6 à 3,5 *Pa-s),* de préférence entre 26 et 30 Poises (2,6 à 3 *Pa·s).*

Le protocole de mesure de la viscosité est le suivant :
La viscosité est mesurée avec un viscosimètre Rhéomat équipé d'un mobile 2, 3 ou 4. Les mesures sont effectuées à une température de 25°C +/- 0.5°C après 10 minutes de rotation du mobile à la vitesse de 200 tours/minute.

Les constituants de la composition selon l'invention sont maintenant décrits plus en détails.

### Phase aqueuse

La composition selon l'invention comprend un milieu aqueux physiologiquement acceptable comprenant au moins de l'éthanol. Par « physiologiquement acceptable », on entend un milieu compatible avec les matières kératiniques.

La composition selon l'invention comprend un milieu aqueux comprenant au moins de l'eau. Le milieu aqueux comprend également au moins de l'éthanol.

Le milieu aqueux peut comprendre au moins un autre solvant organique soluble dans l'eau, à 25°C, choisi par exemple parmi les alcanols, linéaires ou ramifiés, en C3-C4, tels que l'isopropanol, le propanol, le butanol; les polyols ayant notamment de 2 à 20 atomes de carbone, de préférence de 2 à 6 atomes de carbone, comme, le glycérol, le diglycérol, le propylèneglycol, l'isoprène glycol, le dipropylèneglycol, le butylène glycol, l'hexylène glycol, le 1,3-propanediol, le pentylène glycol, les polyéthylèneglycols ayant de 2 à 200 motifs d'oxyde d'éthylène ; et leurs mélanges.

De préférence, le milieu aqueux comprend également au moins un polyol ayant de 2 à 20 atomes de carbone, de préférence de 2 à 6 atomes de carbone.

La composition comprend de préférence de 30 à 55% en poids d'eau par rapport au poids total de la composition, de préférence de 35 à 50%.

De préférence, la composition comprend de 15 à 40% en poids d'éthanol par rapport au poids total de la composition, de préférence de 15 à 30% en poids, de préférence de 15 à 25% en poids.

La quantité d'autre(s) solvant(s) organique(s) peut aller par exemple de 1 à 30% en poids, de préférence de 5 à 25% en poids, mieux de 10 à 20% en poids par rapport au poids total de la composition.

### i) Polymère sulfonique

Les compositions selon l'invention comprennent au moins un polymère sulfonique choisi parmi les homopolymères d'acide acrylamido-2-méthyl propane sulfonique ou ses sels, et les copolymères d'acide acrylamido-2-méthyl propane sulfonique ou ses sels et d'un ou plusieurs monomères non ioniques.

Le polymère sulfonique peut être réticulé ou non réticulé.

Le polymère sulfonique peut avoir un poids moléculaire moyen en nombre allant de 1000 à 20 000 000 g/mole, de préférence allant de 20 000 à 5 000 000 et plus préférentiellement encore de 100 000 à 1 500 000 g/mole.

Les compositions selon l'invention peuvent ainsi comprendre au moins un homopolymère d'acide acrylamido-2-méthyl propane sulfonique ou ses sels. Plus particulièrement, on utilise l'acide 2-acrylamido-2-méthylpropane-sulfonique ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire. On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10% en mole et plus particulièrement de 0,2 à 2% en mole par rapport au polymère.

Lorsque les polymères utilisés sont des homopolymères, ils ne comportent que des monomères à groupement sulfonique et, s'ils sont réticulés, un ou plusieurs agents de réticulation.

Les homopolymères d'acide 2-acrylamido-2-méthylpropane-sulfonique préférés sont en général caractérisés par le fait qu'ils comprennent, distribués de façon aléatoire :
a) de 90 à 99,9% en poids de motifs de formule générale (1) suivante : 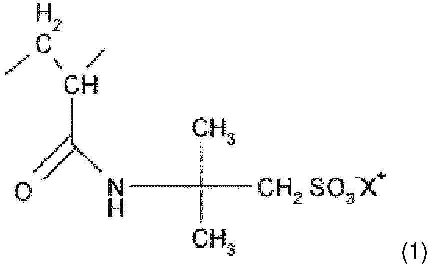 dans laquelle X⁺ désigne un proton, un cation de métal alcalin tel que sodium ou potassium, un cation alcalino-terreux tel que calcium ou l'ion ammonium, au plus 10% mol des cations X⁺ pouvant être des protons H⁺;
b) de 0,01 à 10% en poids de motifs réticulants provenant d'au moins un monomère ayant au moins deux double-liaison oléfiniques, les proportions en poids étant définies par rapport au poids total du polymère.

Les homopolymères selon l'invention plus particulièrement préférés comprennent de 98 à 99,5 % en poids de motifs de formule (1) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS^{®} » (nom CTFA : ammonium polyacryldimethyltauramide).

Le polymère peut être aussi un homopolymère amphiphile (ou homopolymère modifié hydrophobe) choisi parmi les polymères amphiphiles statistiques d'acide 2-acrylamido-2-méthylpropane-sulfonique modifiés par réaction avec une n-monoalkylamine ou une di-n-alkylamine en C6-C22, tels que ceux décrits dans le document WO-A-00/31154, qui sont des homopolymères greffés.

Les compositions selon l'invention peuvent également comprendre au moins un copolymère d'acide acrylamido-2-méthyl propane sulfonique ou ses sels et d'un ou plusieurs monomères non ioniques.

Les copolymères d'AMPS^{®} selon l'invention peuvent être réticulés ou non-réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire. De tels agents sont décrits ci-dessus.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Les copolymères selon l'invention sont obtenus à partir de l'AMPS^{®} et d'un ou plusieurs monomères non ioniques à insaturation éthylénique hydrophiles ou hydrophobes et, s'ils sont réticulés, un ou plusieurs agents de réticulation tels que ceux définis ci-dessus.

Le monomère d'acide 2-acrylamido-2-méthylpropane sulfonique du copolymère contenu dans la composition conforme à l'invention est sous forme libre ou est neutralisé partiellement ou totalement par une base minérale (soude, potasse, ammoniaque) ou une base organique telle que la mono-, di-, ou tri-éthanolamine, un aminométhylpropanediol, la N-méthyl-glucamine, les acides aminés basiques comme l'arginine et la lysine ainsi que le mélange de ces composés.

De préférence, le monomère d'acide 2-acrylamido-2-méthylpropane sulfonique selon l'invention est partiellement ou totalement salifié sous forme de sel d'ammonium ou de sodium.

De préférence, le monomère d'acide 2-acrylamido-2-méthylpropane sulfonique selon l'invention est totalement salifié, de préférence sous forme de sel d'ammonium ou de sodium.

Les copolymères d'AMPS^{®} selon l'invention contiennent un ou plusieurs monomères non ioniques choisis parmi les monomères à insaturation éthylénique hydrosolubles, les monomères hydrophobes, ou leurs mélanges.

Parmi les monomères hydrosolubles non-ioniques, on peut citer par exemple :
- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule CH₂=CHOH,
- les monomères vinyliques hydrosolubles de formule (2) suivante :

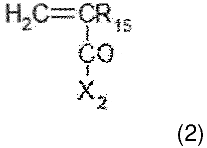

Formule (2) dans laquelle :
- R₁₅ est choisi parmi H, -CH₃, -C₂H₅ ou -C₃H₇
- X₂ est choisi parmi :
- les oxydes d'alkyle de type -OR₁₆ où R₁₆ est un radical hydrocarboné, linéaire ou ramifié, saturé ou insaturé, ayant de 1 à 6 carbones, éventuellement substitué par un atome d'halogène (iode, brome, chlore, fluor); un groupement hydroxy (-OH); éther.

Citons par exemple le (méth)acrylate de glycidyle, le (méth)acrylate d'hydroxyéthyle, et les (méth)acrylates d'éthylène glycol, de diéthylèneglycol ou de polyalkylèneglycol.

De préférence le monomère hydrosoluble est choisi parmi l'acrylamide, la vinylpyrrolidone, les hydroxyalkyl(meth)acrylates, plus particulièrement la vinylpyrrolidone.

Comme copolymères d'AMPS^{®} conformes à l'invention avec des monomères hydrophiles, on peut citer par exemple :
- les copolymères d'acide acrylamido-2-methyl propane sulfonique et de vinylpyrrolidone tels que notamment le produit commercial ARISTOFLEX AVC vendu par CLARIANT,
- les copolymères réticulés acrylamide/acrylamido-2-methyl propane sulfonate de sodium, tels que celui utilisé dans le produit commercial SEPIGEL 305^{®} (nom INCI : Polyacrylamide/C₁₃-C₁₄ Isoparaffin/ Laureth-7) ou celui utilisé dans le produit commercial vendu sous la dénomination dénomination SIMULGEL 600^{®} (nom INCI : Acrylamide / Sodium Acryloyldimethyltaurate / Isohexadecane /Polysorbate-80^{®}) par la société SEPPIC;
- les copolymères d'AMPS^{®} et d'hydroxyéthyl acrylate, comme par exemple le copolymère AMPS^{®} de sodium/hydroxyéthyl acrylate tel que celui utilisé dans le produit commercial vendu sous la dénomination SIMULGEL NS^{®} par la société SEPPIC (nom INCI : Hydroxyethyl acrylate/Sodium Acryloyldimethyltaurate copolymer (and) Squalane (and) Polysorbate 60).

La concentration en homopolymère ou copolymère d'AMPS^{®} (i.e. en matière active) va généralement de 0,05 à 1% en poids par rapport au poids total de la composition, et de préférence de 0,05 à 0,8% en poids, et encore plus particulièrement de 0,1 à 0,5% en poids.

### ii) Copolymère réticulé d'acide acrylique et d'acrylate d'alkyle en C10-C30

La composition selon l'invention comprend au moins un copolymère réticulé d'acide acrylique et d'acrylate d'alkyle en C10-C30.

Le monomère d'acide acrylique est présent de préférence dans des quantités allant de 60 à 95% en poids par rapport au poids total du copolymère.

Le monomère d'acrylate d'alkyle en C10-C30 est présent de préférence dans des quantités allant de 1 à 50% en poids et plus particulièrement de 4 à 40% en poids par rapport au poids total du copolymère.

Le copolymère réticulé d'acide acrylique et d'acrylate d'alkyle en C10-C30 ceux comportant a) au moins un motif hydrophile de type acide carboxylique insaturé oléfinique, et ib) au moins un motif hydrophobe de type ester d'alkyl (C₁₀-C₃₀) d'acide carboxylique insaturé. On peut citer les esters d'alkyles (C₁₀-C₃₀) d'acides carboxyliques de l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle. Des polymères de ce type sont par exemple décrits et préparés, selon les brevets US-3 915 921 et 4 509 949.

Parmi ce type de polymères associatifs, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment. Le copolymère est typiquement partiellement ou totalement réticulé par au moins un agent réticulant classique. Les agents réticulants sont notamment des composés polyinsaturés. Ces composés sont notamment les diallylphtalates, le divinylbenzène, le (méth)acrylate d'allyle, le di(méth)acrylate de (poly)éthylèneglycol ou le méthylène bis-acrylamide. La teneur en agent réticulant varie de 0% à 6% en poids et de préférence de 0,001 à 6% en poids par rapport au poids total du copolymère.

Parmi lesdits polymères ci-dessus, on préfère tout particulièrement les produits vendus par la société LUBRIZOL sous les dénominations commerciales PEMULEN TR1^{®}, PEMULEN TR2^{®}, CARBOPOL 1382^{®}, CARBOPOL ETD 2020^{®}, CARBOPOL ULTREZ 20^{®}, CARBOPOL ULTREZ 21^{®} (nom INCI : Acrylates /C10-30 alkyl acrylate crosspolymer), et encore plus préférentiellement le PEMULEN TR1 et le produit vendu par la société S.E.P.P.I.C. sous la dénomination COATEX SX^{®}, et le CARBOPOL ULTREZ 21.

La concentration en copolymère réticulé (i.e. en matière active) va généralement de 0,05 à 1% en poids par rapport au poids total de la composition, et de préférence de 0,1 à 0,8% en poids, et encore plus particulièrement de 0,2 à 0,6% en poids.

### iii) Polymère épaississant organique supplémentaire

Selon une mode de réalisation particulier de l'invention, la composition comprend en outre iii) un ou plusieurs polymères épaississants organiques différents des polymères i) et ii) tels que définis précédemment.

Par « *polymère épaississant* » on entend un polymère qui, introduit à 1% en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1 s⁻¹. Cette viscosité peut être mesurée à l'aide d'un viscosimètre cône/plan (Rhéomètre Haake R600 ou analogue). Les polymères épaississants peuvent être épaississants de la phase aqueuse et/ou de la phase grasse, préférentiellement de la phase aqueuse.

Par polymère épaississant « *organique* », on entend un polymère épaississant tel que défini précédemment qui est constitué de carbone, d'hydrogène, et éventuellement d'azote, d'oxygène, de soufre, d'halogènes tel que le fluor, le chlore, le brome ainsi que du phosphore, des métaux alcalins tels que le sodium, le potassium, ou métaux alcalino-terreux tels que le magnésium ou le calcium. Les polymères organiques selon l'invention ne comprennent pas de silicium.

Par l'expression « *polymère épaississant organique non cellulosique* », on entend selon l'invention, un polymère épaississant organique ne comportant pas de motif cellulose.

Les polymères épaississants organiques selon l'invention peuvent être d'origine naturelle ou synthétique ; de préférence naturelle.

Les polymères épaississants peuvent être des polymères anioniques, cationiques, amphotères ou non ioniques, associatifs ou non.

Ils peuvent être épaississants des phases aqueuses ou huileuses.

A titre de polymères épaississants de phase aqueuse, on peut citer les polymères épaississants, associatifs ou non associatifs, de préférence non associatif, à motifs sucres.

Par motif « *sucre* » on entend au sens de la présente invention un motif issu d'un carbohydrate de formule Cₙ(H₂O)ₙ₋₁ ou (CH₂O)ₙ pouvant être éventuellement modifié par substitution, et/ou par oxydation et/ou par déshydratation.

Les motifs sucre pouvant entrer dans la composition des polymères épaississants de l'invention sont de préférence issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose.

On peut notamment citer à titre de polymères épaississants de l'invention les gommes natives telles que :
a) les exsudats d'arbres ou d'arbustes dont :
   - la gomme arabique (polymère ramifié de galactose, d'arabinose, de rhamnose et d'acide glucuronique) ;
   - la gomme ghatti (polymère issu d'arabinose, de galactose, de mannose, de xylose et d'acide glucuronique) ;
   - la gomme karaya (polymère issu d'acide galacturonique, de galactose, de rhamnose et d'acide glucuronique) ;
   - la gomme tragacanthe (ou adragante) (polymère d'acide galacturonique, de galactose, de fucose, de xylose et d'arabinose) ;
b) les gommes issues d'algues dont :
   - l'agar (polymère issu de galactose et d'anhydrogalactose) ;
   - les alginates (polymères d'acide mannuronique et d'acide glucuronique) ;
   - les carraghénanes et les furcelleranes (polymères de galactose sulfate et d'anhydrogalactose sulfate) ;
c) les gommes issues de semences ou tubercules dont :
   - la gomme de guar (polymère de mannose et de galactose) ;
   - la gomme de caroube (polymère de mannose et de galactose) ;
   - la gomme de fenugrec (polymère de mannose et de galactose) ;
   - la gomme de tamarin (polymère de galactose, de xylose et de glucose) ;
   - la gomme de konjac (polymère de glucose et mannose) ;
d) les gommes microbiennes dont :
   - la gomme de xanthane (polymère de glucose, de mannose acétate, de mannose/acide pyruvique et d'acide glucuronique) ;
   - la gomme de gellane (polymère de glucose partiellement acylé, de rhamnose et d'acide glucuronique) ;
   - la gomme de scléroglucane (polymère du glucose) ;
e) les extraits de plantes dont :
   - la cellulose (polymère du glucose) ;
   - l'amidon (polymère du glucose) et
   - l'inuline.

Ces polymères peuvent être modifiés par voie physique ou chimique. A titre de traitement physique on peut citer notamment la température.

A titre de traitements chimiques on peut citer les réactions d'estérification, d'étherification, d'amidification, d'oxydation. Ces traitements permettent de conduire à des polymères qui peuvent être notamment non ioniques, anioniques ou amphotères.

De préférence ces traitements chimiques ou physiques sont appliqués sur les gommes de guar, les gommes de caroube, les amidons et les celluloses.

Les gommes de guar non-ioniques utilisables selon l'invention peuvent être modifiées par des groupements (poly)hydroxylakyle en C₁-C₆.

Parmi les groupements (poly)hydroxyalkyle en C₁-C₆, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons peuvent être modifiés par voie chimique ou physique : notamment par une ou plusieurs des réactions suivantes : prégélatinisation, oxydation, réticulation, estérification, éthérification, amidification, traitements thermiques.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les molécules d'amidon peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

Les polymères épaississants non associatifs de l'invention peuvent être des polymères cellulosiques ne comportant pas de chaînes grasse en C₁₀-C₃₀ dans leur structure.

Par polymère « *cellulosique* »*,* on entend selon l'invention tout composé polysaccharidique possédant dans sa structure des enchaînements de résidus glucose unis par des liaisons β-1,4 ; outre les celluloses non substituées, les dérivés de celluloses peuvent être anioniques, cationiques, amphotères ou non-ioniques.

Ainsi, les polymères cellulosiques de l'invention peuvent être choisis parmi les celluloses non substituées y compris sous une forme microcristalline et les éthers de cellulose.

Parmi ces polymères cellulosiques, on distingue les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose...) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques sans chaine grasse en C₁₀-C₃₀ i.e. « *non associatifs* », on peut citer les (C₁-C₄)alkylcelluloses telles que les méthylcelluloses et les éthylcelluloses (par exemple Ethocel standard 100 Premium de DOW CHEMICAL) ; les (poly)hydroxy(C₁-C₄)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques sans chaine grasse, on peut citer les (poly)carboxy(C₁-C₄)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques sans chaine grasse on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy(C₁-C₄)alkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium. Les produits commercialisés répondant à cette définition sont plus particulièrement les produits vendus sous la dénomination "Celquat^{®} L 200" et "Celquat^{®} H 100" par la Société National Starch.

Selon un mode de réalisation particulier de l'invention le ou les polymères épaississant de l'invention sont issus de la (co)polymérisation de monomère acrylate CH₂=C(R')-COOR"' (Vla) et/ou de monomère acrylamide CH₂=C(R')-CO-N(R")-L-Y⁻ M⁺ (VIb) Formules (Via) et (Vlb) dans lesquelles R', et R", identiques ou différents, représentant un atome d'hydrogène ou un groupe (C₁-C₆)alkyle tel que méthyle, de préférence hydrogène, R"' représente un métal alcalin, un métal alcalino-terreux, un atome d'hydrogène ou un groupe (C₁-C₆)alkyle éventuellement substitué notamment par un ou plusieurs groupes hydroxy, carboxy ou amino, de préférence R"' représente un atome d'hydrogène, L représentant un groupe hydrocarboné divalent, cyclique ou acylique, saturé ou insaturé, linéaire ou ramifié, éventuellement interrompu par un ou plusieurs hétéroatomes tels que O, N et comprenant de 1 à 20 atomes de carbones, de préférence de 1 à 6 atomes de carbones, de préférence L représente le groupe divalent -[C(R')(R")]ₚ- avec p représentant un entier compris entre 1 et 4, de préférence 2 et 3 tel que 2, R' et R" étant tels que définis précédemment, plus particulièrement L représente -C(R')(R")-CH₂- ou -CH₂-C(R')(R")- avec R' et R" tels que définis précédemment, de préférence R' et R" représentent un groupe (C₁-C₄)alkyle tel que méthyle ; Y⁻ représente un groupe anionique tel que carboxylate; phosphate, phosphonate, et M⁺ étant un contre ion cationique de préférence métal alcalin tel que sodium, ledit copolymère pouvant se trouver en émulsion directe ou inverse, de préférence inverse. Plus préférentiellement le ou les polymères épaississant de l'invention sont issus de la copolymérisation de monomère acrylate CH₂=C(R')-COOH (Vla) et de monomère acrylamide CH₂=C(R')-CO-N(R")-L-Y⁻ M⁺ (VIb) tels que définis précédemment.

Parmi les polymères épaississants non associatifs sans motifs sucre utilisables, on peut citer les homopolymères d'acrylate d'ammonium ou les copolymères d'acrylate d'ammonium et d'acrylamide seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent être aussi des copolymères d'acide (méth)acryliques réticulés tels que le polymère vendu sous la dénomination AQUA SF1 par la société NOVEON.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom SIMULGEL 600 acrylamide/sodium acryloyldimethyltaurate copolymer isohexadecane et polysorbate 80 commercialisé par SEPPIC, MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

Parmi les polymères épaississants des phases aqueuses, on peut aussi citer les polymères associatifs non cellulosiques bien connus de l'homme de l'art et notamment de nature non ionique, anionique, cationique ou amphotère.

Il est rappelé que les « *polymères associatifs* » sont des polymères capables, dans un milieu aqueux, de s'associer réversiblement entre eux ou avec d'autres molécules.

Leur structure chimique comprend plus particulièrement au moins une zone hydrophile et au moins une zone hydrophobe.

Par « *groupement hydrophobe* »*,* on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs de type anionique, on peut citer :
- ***(a)*** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse, et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC80^{®} et SALCARE SC90^{®} qui sont des émulsions aqueuses à 30% d'un terpolymère réticulé d'acide méthacrylique, d'acrylate d'éthyle et de steareth-10-allyl éther (40/50/10).

On peut également citer le terpolymère acide acrylique/méthacrylate de lauryle/vinylpyrrolidone commercialisé sous l'appelation Acrylidone LM par la Société ISP.
- ***(c)*** les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608^{®} par la société NEWPHASE TECHNOLOGIES
- ***(d)*** les terpolymères acryliques comprenant :
   i) environ 20% à 70% en poids d'un acide carboxylique à insaturation α,β-monoéthylénique [A],
   ii) environ 20 à 80% en poids d'un monomère à insaturation α,β-monoéthylénique non-tensio-actif différent de [A],
   iii) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- ***(e)*** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22^{®} vendu par la société ROHM et HAAS, qui est un terpolymère acide méthacrylique/acrylate d'éthyle/méthacrylate de stéaryle oxyalkyléné.
- ***(f)*** Les polymères amphiphiles comportant au moins un monomère à insaturation éthylènique à groupement sulfonique, sous forme libre ou partiellement ou totalement neutralisée et comprenant au moins une partie hydrophobe. Ces polymères peuvent être réticulés ou non-réticulés. Ils sont de préférence réticulés.

Selon un mode de réalisation particulier de l'invention le ou les polymères iii) sont associatifs, notamment cationiques. On peut citer :
- ***(I)*** Les polyuréthanes associatifs cationiques ;
- ***(II)*** Le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
* un méthacrylate de di(alkyl en C₁-C₄) amino(alkyle en C₁-C₆),
* un ou plusieurs esters d'alkyle en C₁-C₃₀ et de l'acide (méth)acrylique,
* un méthacrylate d'alkyle en C₁₀-C₃₀ polyéthoxylé (20-25 moles de motif oxyde d'éthylène),
* un allyl éther de polyéthylèneglycol/polypropylèneglycol 30/5,
* un méthacrylate d'hydroxy(alkyle en C₂-C₆), et
* un diméthacrylate d'éthylèneglycol.
***- (III)*** les (poly)hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci. Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle. On peut indiquer comme exemples d'alkylhydroxyéthyl-celluloses quaternisées à chaînes grasses en C₈-C₃₀, telles que les produits QUATRISOFT LM 200^{®}, QUATRISOFT LM-X 529-18-A^{®}, QUATRISOFT LM-X 529-18-B^{®} (alkyle en C₁₂) et QUATRISOFT LM-X 529-8^{®} (alkyle en C₁₈) vendus par la société AQUALON, les produits CRODACEL QM^{®}, CRODACEL QL^{®} (alkyle en C₁₂) et CRODACEL QS^{®} (alkyle en C₁₈) vendus par la société CRODA et le produit SOFTCAT SL 100^{®} vendu par la société AQUALON.
***- (IV)*** les Polymères polyvinyllactames cationiques.

De tels polymères sont par exemple décrits dans la demande de brevet WO-00/68282.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

Selon un autre mode de réalisation particulier le ou les polymères iii) sont associatifs amphotères. Ils sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles % et plus particulièrement encore 1,5 à 6 moles %, par rapport au nombre total de moles de monomères.

Des polymères associatifs amphotères selon l'invention sont par exemple décrits et préparés dans la demande de brevet WO 9844012.

Parmi les polymères associatifs amphotères selon l'invention, on préfère les terpolymères acide acrylique / chlorure de (méth)acrylamidopropyl triméthyl ammonium / méthacrylate de stéaryle.

Les polymères associatifs de type non ionique utilisables selon l'invention sont choisis de préférence parmi :
(a) les copolymères de vinyl pyrrolidone et de monomères hydrophobes à chaîne grasse dont on peut citer à titre d'exemple :
   - les produits ANTARON V216^{®} ou GANEX V2160 (copolymère vinylpyrrolidone / hexadécène) vendu par la société I.S.P.
   - les produits ANTARON V220^{®} ou GANEX V220^{®} (copolymère vinylpyrrolidone / eicosène) vendu par la société I.S.P.
(b) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208^{®}.
(c) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
(d) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
(e) les polymères à squelette aminoplaste éther possédant au moins une chaîne grasse, tels que les composés PURE THIX^{®} proposés par la société SUD-CHEMIE.
(f) les celluloses ou leurs dérivés, modifiés par des groupements comportant au moins une chaîne grasse tels que des groupes alkyls, arylalkyls, alkylaryls ou leurs mélanges où les groupes alkyls sont en C₈- et en particulier :
   * les alkylhydroxyéthylcelluloses non-ioniques telles que les produits NATROSOL PLUS GRADE 330 CS et POLYSURF 67 (alkyle en C₁₆) vendus par la société AQUALON
   * les nonoxynylhydroxyéthylcelluloses non-ioniques tels que le produit AMERCELL HM-1500 vendu par la société AMERCHOL ;
   * les alkylcelluloses non-ioniques telles que le produit BERMOCOLL EHM 100 vendu par la société BEROL NOBEL ;
(g) les dérivés de guar associatifs comme les hydroxypropylguars modifiés par une chaîne grasse tel que le produit ESAFLOR HM 22 (modifié par une chaîne alkyle en C₂₂) vendu par la société LAMBERTI ; le produit MIRACARE XC 95-3 (modifié par une chaîne alkyle en C₁₄) et le produit RE 205-146 (modifié par une chaîne alkyle en C₂₀) vendus par RHODIA CHIMIE ;

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205^{®} à fonction urée vendu par la société RHEOX ou encore les Rhéolates^{®} 208, 204 ou 212, ainsi que l'Acrysol RM 184^{®}.

On peut également citer le produit ELFACOS T210^{®} à chaîne alkyle en C₁₂-C₁₄ et le produit ELFACOS T212^{®} à chaîne alkyle en C₁₈ de chez AKZO.

Le produit DW 1206B^{®} de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le RHEOLATE^{®} 255, le RHEOLATE^{®} 278 et le RHEOLATE^{®} 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations ACULYN 46^{®} et ACULYN 44^{®} [l'ACULYN 46^{®} est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44^{®} est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut aussi utiliser des polymères épaississants des phases grasses.

De préférence, les polymères structurant la phase huileuse via des interactions physiques sont choisis parmi les polyamides, les polyamides siliconés, les mono- ou polyalkylesters de saccharide ou polysaccharide, les dérivés amides d'aminoacides N-acylés, copolymères comprenant une séquence alkylène ou styrène, ces copolymères pouvant être des polymères di-blocs, tri-bloc, multi-bloc, radial-bloc encore appelés copolymères en étoile, ou encore des polymères en peigne.

### 1) Les polymères portant dans le squelette au moins une séquence cristallisable

Il s'agit encore de polymères solubles ou dispersibles dans l'huile ou phase huileuse par chauffage au-dessus de leur point de fusion pF. Ces polymères sont notamment des copolymères séquencés constitués d'au moins 2 séquences de nature chimique différente dont l'une est cristallisable.

A titre de polymères portant dans le squelette au moins une séquence cristallisable convenant à la mise en œuvre de l'invention, on peut mentionner :
i). Les polymères définis dans le document US-A-5,156,911 ;
ii). Les copolymères séquencés d'oléfine ou de cyclooléfine à chaîne cristallisable comme ceux issus de la polymérisation séquencée de :
   - cyclobutène, cyclohexène, cyclooctène, norbornène (c'est-à-dire bicyclo(2,2,1)heptène 2), 5-méthylnorbornène, 5-éthylnorbornène, 5,6-diméthylnorbornène, 5,5,6-triméthyl norbornène, 5-éthylidène-norbornène, 5-phénylnorbornène, 5-benzylnorbornène, 5-vinylnorbornène, 1,4,5,8-diméthano-1,2,3,4,4a,5,8a-octahydronaphtalène, dicyclopentadiène, et leurs mélanges ;
   - avec l'éthylène, le propylène, le 1-butène, le 3-méthyl-1-butène, le 1-hexène, le 4-méthyl-1-pentène, le 1-octène, le 1-décène, le 1-éicosène ou leurs mélanges. Ces copolymères séquencés peuvent être en particulier les copolymères (éthylène/norbornène) blocs et les terpolymères blocs (éthylène/propylène/ éthylidène-norbornène).

On peut aussi utiliser ceux résultants de la copolymérisation séquencée d'au moins 2 α-oléfines en C₂-C₁₆ et mieux en C₂-C₁₂, tels que ceux cités précédemment et en particulier les bipolymères séquencés d'éthylène et d'1-octène.

Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
a) les copolymères séquencés poly(δ-caprolactone)-b-poly(butadiène), utilisés de préférence hydrogénés, tels que ceux décrits dans l'article « Melting behavior of poly(δ-caprolactone)-block-polybutadiène copolymers » de S. Nojima, Macromolécules, 32, 3727-3734 (1999).
b) les copolymères séquencés poly(butylènetéréphtalate)-b-poly(isoprène) hydrogénés séquencés ou multiséquencés, cités dans l'article « Study of morphological and mechanical properties of PP/PBT » de B. Boutevin et al., Polymer Bulletin, 34, 117-123 (1995).
c) les copolymères séquencés poly(éthylène)-b-copoly(éthylène/propylène) cités dans les articles « Morphology of semi-crystalline block copolymers of ethylene- (ethylene-alt-propylene) » de P. Rangarajan et al., Macromolecules, 26, 4640-4645 (1993), et « Polymer agregates with crystalline cores : the system poly(ethylene)- poly(ethylene-propylene) », P. Richter et al., Macromolécules, 30, 1053-1068 25 (1997).
d) les copolymères séquencés poly(éthylène)-b-poly(éthyléthylène) cités dans l'article général « Cristallization in block copolymers » de I.W. Hamley, Advances in Polymer Science, vol 148, 113-137 (1999).

Les polymères semi-cristallins utilisables dans le cadre de l'invention peuvent être non réticulés ou réticulés en partie, du moment que le taux de réticulation ne gêne pas leur dissolution ou dispersion dans la phase huileuse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins convenant à l'invention sont non réticulés.

A titre d'exemple particulier de polymère semi-cristallin utilisable dans la composition selon l'invention, on peut citer les produits Intelimer^{®} de la société Landec décrits dans la brochure « Intelimer^{®} polymers ». Ces polymères sont sous forme solide à température ambiante (25 °C). Ils sont porteurs de chaînes latérales cristallisables et présentent le monomère. On peut citer notamment le « Landec IP22^{®} », ayant une température de fusion pF de 56 °C, qui est un produit visqueux à température ambiante, imperméable, non-collant.

On peut aussi utiliser les polymères semi-cristallins décrits dans les exemples 3, 4, 5, 7, 9 du document US-A-5,156,911, résultant de la copolymérisation d'acide acrylique et d'alkyl(méth)acrylate en C₅ à C₁₆ comme ceux résultant de la copolymérisation :
- d'acide acrylique, d'hexadécylacrylate et d'isodécylacrylate dans un rapport 1/16/3,
- d'acide acrylique et de pentadécylacrylate dans un rapport 1/19,
- d'acide acrylique, d'hexadécylacrylate, d'éthylacrylate dans un rapport 2,5/76,5/20,
- d'acide acrylique, d'hexadécylacrylate et de méthylacrylate dans un rapport 5/85/10,
- d'acide acrylique, d'octadécylméthacrylate dans un rapport 2,5 / 97,5.

On peut aussi utiliser le polymère « Structure O » commercialisé par la société National Starch, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP ou par copolymérisation de l'acrylate de béhényle et de l'acide acrylique ou de NVP, tels que décrits dans le document US-A-5,519,063 ou EP-A- 0 550 745.

Selon une variante particulière de réalisation, les polymères semi-cristallins convenant à la mise en œuvre de la présente invention sont notamment les acrylates alkylés, parmi lesquels on peut mentionner les copolymères de LANDEC :
- Doresco IPA 13-1^{®} : poly acrylate de stéaryle, pF de 49 °C et PM de 145000;
- Doresco IPA 13-3^{®} : poly acrylate/acide méthacrylique, pF de 65 °C et PM de 114000;
- Doresco IPA 13-4^{®} : poly acrylate/vinyl pirrolidone, pF de 44 °C et PM de 387000;
- Doresco IPA : poly acrylate/methacrylate d'hydroxyethyle, pF de 47 °C et PM de 397600;
- Doresco IPA 13-6^{®} : poly acrylate de béhényle, pF de 66 °C.

### 2) Les polyamides non siliconés

Les polyamides particuliers utilisés dans la composition selon la présente invention sont de préférence ceux décrits dans le document US-A-5,783,657 de la Société UNION CAMP. La partie de US-A-5,783,657 consacrée à ces polymères est incorporée par référence.

Chacun de ces polyamides satisfait notamment à la formule (V) suivante :

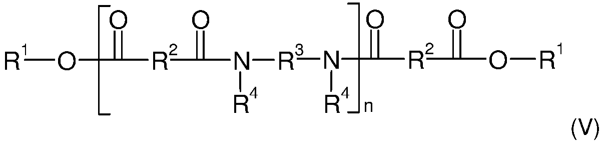

Formule (V) dans laquelle :
- n désigne un nombre entier de motifs amide tel que le nombre de groupes ester représente de 10 % à 50 % du nombre total des groupes ester et amide ;
- R¹ est à chaque occurrence indépendamment un groupe alkyle ou alcényle ayant au moins 4 atomes de carbone et notamment de 4 à 24 atomes de carbone
- R² représente à chaque occurrence indépendamment un groupe hydrocarboné en C₄ à C₅₅ à condition que 50 % au moins des groupes R₂ représentent un groupe hydrocarboné en C₃₀ à C₅₅ ;
- R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et
- R⁴ représente à chaque occurrence indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure hétérocyclique définie par R₄-N-R₃, avec au moins 50 % des R₄ représentant un atome d'hydrogène.

En particulier, les groupes ester de ce polyamide représentent de 15 à 40 % du nombre total des groupes ester et amide et au mieux de 20 à 35 %. De plus, n représente avantageusement un nombre entier allant de 1 à 10, et mieux de 1 à 5, bornes incluses.

De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % au moins des R² sont des groupes ayant de 30 à 42 atomes de carbone. Les autres R² sont des groupes hydrogénés en C₄ à C₁₉ et de préférence en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

L'épaississement de la phase grasse liquide peut être obtenue à l'aide d'un ou plusieurs polyamides définis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Comme polyamide structurant utilisable dans l'invention, on peut encore citer les résines polyamides résultant de la condensation d'un acide di-carboxylique aliphatique et d'une diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque Versamid^{®} par les sociétés General Mills, Inc. et Henkel Corp., sous la marque Onamid^{®} notamment Onamid S ou C. Ces résines ont une masse moléculaire moyenne en poids allant de 6000 et 9000. Pour plus d'information sur ces polyamides, on peut se référer aux documents US-A-3,645,705 et US-A-3,148,125. Plus spécialement, on utilise les Versamid^{®} 30 ou 744.

### 2) Les mono- ou polyalkylesters de saccharide ou polysaccharide

Parmi les mono ou polyalkylesters de saccharide ou de polysaccharide convenant à la mise en œuvre de l'invention, on peut mentionner les alkyles ou polyalkylesters de dextrine ou d'inuline.

Il peut s'agir notamment d'un mono-ou poly-ester de dextrine et d'au moins un acide gras et notamment répondant à la formule (VI) suivante :

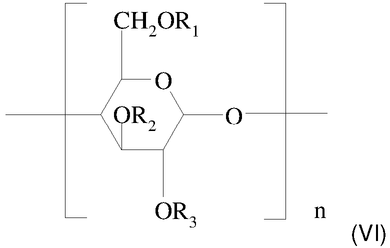

Formule (VI) dans laquelle :
- n est un entier allant de 3 à 200, notamment allant de 20 à 150, et en particulier allant de 25 à 50,
- R₁, R₂ et R₃, identiques ou différents, sont choisis parmi l'hydrogène ou un groupement acyle (R-C(O)-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux R₁, R₂ ou R₃ est différent de l'hydrogène.

En particulier, R₁, R₂ et R₃ peuvent représenter l'hydrogène ou un groupement acyle (R-C(O)-) dans lequel R est un radical hydrocarboné tel que défini précédemment, sous réserve qu'au moins deux desdits radicaux R₁, R₂ ou R₃ sont identiques et différents de l'hydrogène.

L'ensemble des radicaux R₁, R₂ et R₃ peut figurer un groupement acyle (R-C(O)) identique ou différent, et notamment identique.

En particulier, n précédemment exposé varie avantageusement de 25 à 50, notamment est égal à 38 dans la formule générale de l'ester de saccharide utilisable dans la présente invention.

Notamment lorsque les radicaux R₁, R₂ et/ou R₃, identiques ou différents figurent un groupement acyle (R-C(O)), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine, en particulier des palmitates de dextrine, sont disponibles commercialement sous la dénomination RHEOPEARL TL ou RHEOPEARL KL de la société Chiba Flour.

### 3) les dérivés amides d'aminoacides N-acylés

Les amides d'aminoacides N-acylés utilisables sont par exemple les diamides de l'association d'un acide N-acylamine avec des amines comprenant de 1 à 22 atomes de carbone tels que ceux décrits dans le document FR 2 281 162. Ce sont par exemple les dérivés amides d'acide alcoyle glutamique tels que le dibutylamide d'acide laurylglutamique, commercialisé par la société Ajinomoto sous le nom « Gelling agent GP-1 » ou encore le dibutylamide d'acide 2-éthylhexanoyl glutamique commercialisé par la société Ajinomoto sous la dénomination de « Gelling agent GA-01 ».

Parmi les polymères épaississants pour phase grasse les polymères portant dans le squelette au moins une séquence cristallisable sont préférés.

Les polymères épaississants pour phase aqueuse ou phase grasse peuvent être utilisés seuls ou en mélanges en toutes proportions.

De préférence les épaississants sont des épaississants de phase aqueuse.

De préférence, les polymères des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 s-1.

Selon une variante avantageuse la composition de l'invention comprend un ou plusieurs polymères épaississants associatif ou non associatifs, particulièrement A) à motifs sucres en particulier issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose, telle que a) la gomme arabique (polymère ramifié de galactose, d'arabinose, de rhamnose et d'acide glucuronique) ; b) les carraghénanes et les furcelleranes (polymères de galactose sulfate et d'anhydrogalactose sulfate), c) la gomme de guar (polymère de mannose et de galactose) ; d) la gomme de xanthane (polymère de glucose, de mannose acétate, de mannose/acide pyruvique et d'acide glucuronique) ; e) la gomme de gellane (polymère de glucose partiellement acylé, de rhamnose et d'acide glucuronique) ; f) la gomme de scléroglucane (polymère du glucose) ; g) la cellulose (polymère du glucose) ; h) l'amidon (polymère du glucose) ; i) les éthers de cellulose non ioniques sans chaine grasse en C₁₀-C₃₀; B) les polymères issus de la (co)polymérisation de monomère acrylate CH₂=C(R')-COOR"' (VIa) et/ou de monomère acrylamide CH₂=C(R')-CO-N(R")-L-Y⁻ M⁺ (VIb) Formules (Via) et (VIb) dans lesquelles R', et R", identiques ou différents, représentant un atome d'hydrogène ou un groupe (C₁-C₆)alkyle tel que méthyle, de préférence hydrogène, R"' représente un métal alcalin, un métal alcalino-terreux, un atome d'hydrogène ou un groupe (C₁-C₆)alkyle éventuellement substitué notamment par un ou plusieurs groupes hydroxy, carboxy ou amino, de préférence R"' représente un atome d'hydrogène, L représentant un groupe hydrocarboné divalent, cyclique ou acylique, saturé ou insaturé, linéaire ou ramifié, éventuellement interrompu par un ou plusieurs hétéroatomes tels que O, N et comprenant de 1 à 20 atomes de carbones, de préférence de 1 à 6 atomes de carbones, de préférence L représente le groupe divalent -[C(R')(R")]ₚ- avec p représentant un entier compris entre 1 et 4, de préférence 2 et 3 tel que 2, R' et R" étant tels que définis précédemment, plus particulièrement L représente -C(R')(R")-CH₂- ou -CH₂-C(R')(R")- avec R' et R" tels que définis précédemment, de préférence R' et R" représentent un groupe (C₁-C₄)alkyle tel que méthyle ; Y⁻ représente un groupe anionique tel que carboxylate; phosphate, phosphonate, et M⁺ étant un contre ion cationique de préférence métal alcalin tel que sodium,; k) les homopolymères d'acrylate d'ammonium ou les copolymères d'acrylate d'ammonium et d'acrylamide ; C) les « *polymères associatifs* » notamment les copolymères comportant parmi leurs monomères un acide carboxylique à insaturation α,β-monoéthylénique et un ester d'acide carboxylique à insaturation α,β-monoéthylénique et d'un alcool gras oxyalkyléné ; D) les (poly)hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci ; les radicaux alkyle portés par les celluloses ou hydroxyéthylcelluloses quaternisées ci-dessus comportent de préférence de 8 à 30 atomes de carbone, E) les celluloses ou leurs dérivés, modifiés par des groupements comportant au moins une chaîne grasse tels que des groupes alkyls, arylalkyls, alkylaryls ou leurs mélanges où les groupes alkyls sont en C₈- et en particulier les alkylhydroxyéthylcelluloses non-ioniques ; F) les dérivés de guar associatifs comme les hydroxypropylguars modifiés par une chaîne grasse.

De préférence le ou les polymères épaississants organiques iii) est ou sont présents dans la composition selon l'invention dans une quantité allant de 0,01 à 10 % en poids par rapport au poids total de la composition; plus préférentiellement de 0,1 à 5 % en poids par rapport au poids total de la composition et encore plus préférentiellement 0,01 et 2,5 % en poids par rapport au poids total de la composition.

### iv) Tensioactif

Selon une mode de réalisation particulier de l'invention, la composition comprend en outre iv) un ou plusieurs tensioactifs, ils peuvent être anioniques, zwitterioniques ou amphotères, ou non ioniques, de préférence non ioniques ou anioniques, plus préférentiellement non ioniques.

Par « *tensioactif* » on entend un « *agent de surface* » ou « *surfactant* » qui est un composé susceptible de modifier la tension superficielle entre deux surfaces, les tensioactifs sont des molécules amphiphiles, i.e. qui présentent deux parties de polarité différente, l'une lipophile et apolaire et l'autre hydrophile et polaire.

Parmi les tensioactifs « non ioniques » selon l'invention on peut citer, seuls ou mélanges, les alcools gras, les alpha-diols, les alkylphénols ces 3 types composés étant polyéthoxylés, polypropoxylés et/ou polyglycérolés, et ayant une chaîne grasse comportant par exemple 8 à 22 atomes de carbone, le nombre de groupements oxyde d'éthylène ou oxyde de propylène pouvant aller notamment de 2 à 50 et le nombre de groupements glycérol pouvant aller notamment de 2 à 30.

Par « *tensioactif anionique* », on entend un tensioactif ne comportant à titre de groupements ioniques ou ionisables que des groupements anioniques. Ces groupements anioniques sont choisis de préférence parmi les groupements -C(O)OH, -C(O)O⁻, -SO₃H, -S(O)₂O⁻, -OS(O)₂OH, -OS(O)₂O⁻, -P(O)OHé, -P(O)₂O⁻, -P(O)O₂⁻, -P(OH)₂, =P(O)OH, -P(OH)O⁻, =P(O)O⁻, =POH, =PO⁻, les parties anioniques comprenant un contre ion cationique tel que un metal alcalin, un métal alcalino-terreux, ou un ammonium.

A titre d'exemples d'agents tensioactifs anioniques utilisables dans la composition selon l'invention, on peut citer les alkyl sulfates, les alkyl éther sulfates, les alkylamidoéthersulfates, les alkylarylpolyéthersulfates, les monoglycéride-sulfates, les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les acyllactylates, les sels d'acides D-galactoside-uroniques, les sels d'acides alkyl éther-carboxyliques, les sels d'acides alkyl aryl éther-carboxyliques, les sels d'acides alkyl amidoéther-carboxyliques, et les formes non salifiées correspondantes de tous ces composés, les groupes alkyle et acyle de tous ces composés comportant de 6 à 24 atomes de carbone et le groupe aryle désignant un groupe phényle.

Le ou les agents tensioactifs amphotères ou zwittérioniques utilisables dans la présente invention, peuvent être notamment des dérivés d'amines aliphatiques secondaires ou tertiaires éventuellement quaternisées contenant au moins un groupe anionique tel que, par exemple, un groupe carboxylate, sulfonate, sulfate, phosphate ou phosphonate, et dans lesquels le groupe aliphatique ou au moins l'un des groupes aliphatiques est une chaîne linéaire ou ramifiée comportant de 8 à 22 atomes de carbone.

On peut citer en particulier les alkyl(C₈-C₂₀)bétaïnes, les sulfobétaïnes, les (alkyl en C₈-C₂₀)amido(alkyl en C₂-C₈)bétaïnes ou les (alkyl en C₈-C₂₀)amido(alkyl en C₂-C₈)sulfobétaïnes.

La quantité de tensioactifs (de préférence non ioniques ou anioniques, plus préférentiellement non ioniques) va de préférence de 0,05 % à 25 % en poids, en particulier de 0,1 % à 20 % en poids, plus particulièrement de 1 à 10% et encore plus particulièrement de 2 à 5 % en poids rapportée au poids total de la composition de l'invention.

### pH de la composition

De préférence, la composition selon l'invention présente un pH de 5,0 à 6,0. Avantageusement, le pH de la composition est compris entre 5,5 et 5,9.

Selon un mode de réalisation, la composition cosmétique selon l'invention peut comprendre un acide et une base.

Selon une variante, la composition selon l'invention peut comprendre au moins une base.

La base est notamment utilisée pour augmenter le pH de la solution aqueuse initiale. Elle peut être également utilisée pour ajuster le pH final de la composition entre 5,0 et 6,0, de préférence entre 5,5 et 5,9.

La base peut être choisie parmi les bases minérales comme par exemple les hydroxydes de métaux alcalins, l'hydroxyde de sodium, l'hydroxyde de potassium, les hydroxydes d'ammonium, l'ammoniaque, les bases organiques comme par exemple la monoéthanolamine, la diéthanolamine, la tiéthanolamine, la triisopropylamine, la tri[(2-hydroxy) 1 -propyl)] amine, la N,N-diméthyl éthanolamine, le 2-amino 2-méthyl 1-propanol, le 2-amino 2-méthyl 1,3-propanediol, la triéthylamine, la diméthylaminopropylamine et les bases amphotères (c'est-à-dire des bases ayant à la fois des groupements fonctionnels anioniques et cationiques) comme, les amines organiques primaires, secondaires, tertiaires ou cycliques, les acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine et l'un quelconque de leurs mélanges.

Selon un mode de réalisation particulier, la base de la composition est choisie parmi l'hydroxyde de sodium, l'hydroxyde de potassium, les hydroxydes d'ammonium, l'ammoniaque, la monoéthanolamine, la diéthanolamine, la triéthanolamine, la trometamine et l'un quelconque de leurs mélanges. Selon un mode de réalisation particulier, la base de la composition est choisie parmi l'hydroxyde de sodium, la triéthanolamine, et leur mélange.

Selon un mode de réalisation particulier, la base de la composition selon l'invention est présente à une concentration massique inférieure à 0,5%, voire inférieure à 0,25% en masse par rapport à la masse totale de la composition.

Selon une variante, la composition selon l'invention peut comprendre au moins un acide. Il peut être utilisé pour ajuster le pH final de la composition entre 5,0 et 6,0, de préférence entre 5,5 et 5,9.

L'acide peut être choisi parmi les acides minéraux comme l'acide chlorhydrique, l'acide sulfurique, l'acide nitrique, les acides organiques comme l'acide acétique, l'acide lactique, l'acide glycolique, l'acide mandélique, l'acide citrique, l'acide ascorbique et l'un quelconques de leurs mélanges.

L'acide peut être choisi parmi les acides organiques, comme l'acide stéarique, l'acide palmitique, l'acide myristique et l'un quelconque de leurs mélanges.

Selon un mode de réalisation particulier, l'acide de la composition selon l'invention est présent à une concentration massique inférieure à 0,5%, voire inférieure à 0,25% en masse par rapport à la masse totale de la composition.

### Substance parfumante

La composition selon l'invention comprend au moins 5% en poids par rapport au poids total de composition, d'au moins une substance parfumante.

Par « *substance parfumante* », on entend tout parfum ou arôme susceptible de dégager une odeur agréable.

Les parfums sont des compositions contenant notamment les matières premières décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits naturels (huiles essentielles, absolus, résinoïdes, résines, concrètes) et/ou synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

De préférence, la substance parfumante comprend au moins une huile essentielle.

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage (Expression à froid). L'huile essentielle est le plus souvent séparée de la phase aqueuse par un procédé physique n'entraînant pas de changement significatif de la composition.

### Modes d'obtention des huiles essentielles

Le choix de la technique dépend principalement de la matière première : son état originel et ses caractéristiques, sa nature proprement dit. Le rendement « huile essentielle/matière première végétale » peut être extrêmement variable selon les plantes : 15 ppm à plus de 20%. Ce choix conditionne les caractéristiques de l'huile essentielle, en particulier viscosité, couleur, solubilité, volatilité, enrichissement ou appauvrissement en certains constituants.

### Entraînement à la vapeur d'eau

L'entrainement à la vapeur correspond à la vaporisation en présence de vapeur d'eau d'une substance peu miscible à l'eau. La matière première est mise en présence d'eau portée à ébullition ou de vapeur d'eau dans un alambic. La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. On appelle « eau aromatique ou « hydrolat » ou « eau distillée florale », le distillat aqueux qui subsiste à l'entraînement à la vapeur d'eau, une fois la séparation de l'huile essentielle effectuée.

### Distillation sèche

L'huile essentielle est obtenue par distillation des bois, écorces ou racines, sans addition d'eau ou de vapeur d'eau dans une enceinte fermée conçue pour que le liquide soit récupéré dans sa partie basse. L'huile de Cade constitue l'exemple le plus connu de ce mode d'obtention.

### Expression à froid

Ce mode d'obtention ne s'applique qu'aux fruits agrumes (Citrus spp) par des procédés mécaniques à température ambiante. Le principe de la méthode est le suivant : les zestes sont dilacérés et le contenu des poches sécrétrices qui ont été rompues est récupéré par un procédé physique. Le procédé classique consiste à exercer sous un courant d'eau une action abrasive sur toute la surface du fruit. Après élimination des déchets solides, l'huile essentielle est séparée de la phase aqueuse par centrifugation. La plupart des installations industrielles permettent en fait la récupération simultanée ou séquentielle des jus de fruits et de l'huile essentielle.

### Caractères physico-chimiques

Les huiles essentielles sont en général volatiles et liquides à température ambiante, ce qui les différencie des huiles dites fixes. Elles sont plus ou moins colorées et leur densité est en général inférieure à celle de l'eau. Elles ont un indice de réfraction élevée et la plupart dévient la lumière polarisée. Elles sont liposolubles et solubles dans les solvants organiques usuels, entraînables à la vapeur d'eau, très peu solubles dans l'eau.

Parmi les huiles essentielles utilisables selon l'invention, on peut citer celles obtenues à partir des plantes appartenant aux familles botaniques suivantes :
Abiétaceés ou Pinacées : conifères
Amaryllidacées
Anacardiacées
Anonacées : ylang
Apiacées (par exemple les ombellifères) : aneth, angénique, coriandre, criste marine, carotte, persil
Aracées
Aristolochiacées
Astéracées : achilée, armoise, camomille, hélichryse
Bétulacées
Brassicacées
Burséracées : encens
Caryophyllacées
Canellacées
Césalpiniacées : copaïfera (copahu)
Chénopodacées
Cistacées : ciste
Cypéracées
Diptérocarpacées
Ericacées : gaulthérie (wintergreen)
Euphorbiacées
Fabacées
Geraniacées : géranium
Guttifères
Hamamélidacées
Hernandiacées
Hypéricacées : millepertuis
Iridacées
Juglandacées
Lamiacées : thym, origan, monarde, sarriette, basilic, marjolaines, menthes, patchouli, lavandes, sauges, cataire, romarin, hysope, mélisse, romarin
Lauracées : ravensara, laurier, bois de rose, cannelle, litséa
Liliacées : ail
Magnoliacées : magnolia
Malvacées
Méliacées
Monimiacées
Moracées : chanvre, houblon
Myricacées
Mysristicacées : muscade
Myrtacées : eucalyptus, tea tree, niaouli, cajeput, backousia, girofle, myrte
Oléacées
Pipéracées : poivre
Pittosporacées
Poacées : citronnelle, lemongrass, vétiver
Polygonacées
Renonculacées
Rosacées : roses
Rubiacées
Rutacées : tous les citrus
Salicacées
Santalacées : santal
Saxifragacées
Schisandracées
Styracacées : benjoin
Thymélacées : bois d'agar
Tilliacées
Valérianacées : valériane, nard
Verbénacées : lantana, verveine
Violacées
Zingibéracées : galanga, curcuma, cardamome, gingembre
Zygophyllacées.

On peut citer également les huiles essentielles extraites de fleurs (lis, lavande, rose, jasmin, ylang-ylang, néroli), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes ( galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol, l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha -hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indanes, les muscs-tétralines, les muscs-isochromanes, les cétones macrocycliques, les muscs-macrolactones, le brassylate d'éthylène, les muscs aliphatiques et leurs mélanges.

Selon un mode préféré de réalisation de l'invention, on utilise un mélange de différentes substances parfumantes qui engendrent en commun une note plaisante pour l'utilisateur.

On choisira de préférence les substances parfumantes de telle sorte qu'elles produisent des notes (tête, cœur et fond) dans les familles suivantes :
les hespéridés,
les aromatiques,
les notes florales en particulier fleurs roses et fleurs blanches,
les épicées,
les boisées,
les gourmands,
les chyprés,
les fougères,
les cuirés,
les muscs.

Les compositions parfumantes de l'invention contiennent de préférence de 5% à 30% en poids de substance parfumante, mieux de 10% à 25% en poids, en particulier de 15 à 25% en poids par rapport au poids total de la composition.

Selon un mode de réalisation particulier, la composition parfumante selon l'invention comprend en outre des actifs et/ou des excipients cosmétiquement acceptables.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

De préférence, la composition parfumante selon l'invention comprend moins de 2% en poids d'huile par rapport au poids total de la composition, de préférence moins de 1% en poids d'huile, de préférence moins de 0,5% en poids d'huile. De préférence, la composition parfumante selon l'invention est exempte d'huile. L'huile est ici distincte de la substance parfumante. Par « huile », on entend tout corps gras non aqueux liquide à température ambiante (25°C) et pression atmosphérique (760 mm de Hg), et différent de la substance parfumante. En particulier l'huile est ici distincte des huiles essentielles.

De préférence, la composition parfumante selon l'invention consiste en:
un milieu aqueux physiologiquement acceptable comprenant au moins de l'éthanol ;
au moins un polymère sulfonique choisi parmi les homopolymères d'acide acrylamido-2-méthyl propane sulfonique ou ses sels et les copolymères d'acide acrylamido-2-méthyl propane sulfonique ou ses sels et d'un ou plusieurs monomères non ioniques ;
au moins un copolymère réticulé d'acide acrylique et d'acrylate d'alkyle en C10-C30 ; et
au moins 5% en poids par rapport au poids total de composition, d'au moins une substance parfumante.

L'invention se rapporte également à une méthode de soin cosmétique et/ou esthétique, notamment pour parfumer, des matières kératiniques comprenant l'application topique sur les matières kératiniques, de préférence la peau, d'une composition selon l'invention.

Des exemples concrets, mais nullement limitatifs, illustrant l'invention, vont maintenant être donnés.

Dans les exemples, la température est celle ambiante (20°C) et exprimée en degré Celsius sauf indication contraire, et la pression est la pression atmosphérique, sauf indication contraire.

Dans les exemples, les quantités des ingrédients des compositions sont données en % en poids par rapport au poids total de composition.

### Exemple 1 : Composition parfumante selon l'invention et compositions comparatives

I/ La composition selon l'invention selon le Tableau 1 est préparée selon le procédé suivant :
   On mélange l'eau, l'alcool (éthanol) et les polyols à température ambiante sous agitation au Moritz ;
   On ajoute le Carbopol Ultrez 21 Polymer et on agite ;
   On ajoute le parfum ;
   Enfin, on ajoute le Sepigel 305 puis la base.

**[Tableau 1]**

| **Ingrédients** | **Quantité (% en poids par rapport au poids total de composition) Composition selon l'invention** |
|---|---|
| 2-AMINO-2-METHYL-1-PROPANOLAMINOMETHYL PROPANOL | 0,06 |
| Parfum | 20 |
| Copolymère acrylamide/acrylamido-2-méthyl propane sulfonate de sodium en émulsion inverse à 40% dans isoparaffine/eau (Sepigel 305 de chez Seppic) | 0,5 |
| Copolymère réticulé acide acrylique/methacrylate d'alkyl C₁₀-C₃₀ (CARBOPOL ULTREZ 21 POLYMER de chez Lubrizol) | 0,4 |
| GLYCERINE | 2 |
| Ethanol | 21 |
| DIPROPYLENE GLYCOL | 15 |
| Eau | Qsp 100 |

Cette composition selon l'invention a un pH de 5,67 et une viscosité de 28 poises (2,8 Pa·s) mesurée selon le protocole décrit précédemment. Lorsque la composition est conservée à 4°C pendant un mois, elle a un pH de 5,5 et une viscosité de 26 poises (2,6 Pa·s) mesurée selon le protocole décrit précédemment.

Cette composition comprend 20% en poids de concentré de parfum, dans un gel hydroalcoolique. Elle contient aussi 21% en poids d'éthanol. Elle est cosmétiquement intéressante, car elle présente un fini poudré, a une prise agréable et facile, et est très fraîche et agréable à l'application. La composition est stable et homogène même après un stockage d'un mois à une large gamme de température : 4 °C ou à 45 °C.

Il/ Les compositions comparatives A à D selon le Tableau 2 sont préparées selon le même procédé que décrit pour la composition selon l'invention, mais sans Sepigel 305 et/ou sans Carbopol Ultrez 21. Pour la composition comparative C, l'Aristoflex SNC est introduit de la même façon que le Sepigel 305.

**[Tableau 2]**

| Ingrédients | Quantité (% en poids par rapport au poids total de composition) Formule A | Quantité (% en poids par rapport au poids total de composition) Formule B | Quantité (% en poids par rapport au poids total de composition) Formule C | Quantité (% en poids par rapport au poids total de composition) Formule D |
|---|---|---|---|---|
| 2-AMINO-2-METHYL-1-PROPANOLAMINOMETH YL PROPANOL | 0,06 | 0,06 | 0,06 | 0,06 |
| Parfum | 20 | 20 | 20 | 20 |
| COPOLYMERE AMPS / METHACRYLATE D'ALCOOL C16/C18 ETHOXYLE (8 MOLES EO) 80 / 20 à 92 % dans un mélange eau/butanol (5/3) (Aristoflex SNC de Clariant) | - | - | 0,5 | - |
| Copolymère acrylamide/acrylamido 2-méthyl propane sulfonate de sodium en émulsion inverse à 40% dans isoparaffine/eau (Sepigel 305 de chez Seppic) | - | 0,5 | - | 0,9 |
| Copolymère réticulé acide acrylique/methacrylate d'alkyl C₁₀-C₃₀ (CARBOPOL ULTREZ 21 POLYMER de chez Lubrizol) | 0,4 | - | - | - |
| GLYCERINE | 2 | 2 | 2 | 2 |
| Ethanol | 21 | 21 | 21 | 21 |
| DIPROPYLENE GLYCOL | 15 | 15 | 15 | 15 |
| Eau | Qsp 100 | Qsp 100 | Qsp 100 | Qsp 100 |

La formule comparative A a un pH de 6 et une viscosité de 20 poises (2 Pa·s) mesurée selon le protocole décrit précédemment. Elle est épaisse, elle a une texture plus aqueuse et est moins facile à prendre que la formule selon l'invention.

La formule comparative B a un pH de 5,6 et une viscosité de 12 poises (1,2 Pa·s) mesurée selon le protocole décrit précédemment. Elle est très fluide, a une texture plus collante et est moins fraîche que la formule selon l'invention.

La formule comparative C, elle casse dès T0. Elle est donc instable.

Quant à la formule comparative D, elle est bien trop fluide et difficile à la prise. Elle a un pH de 5,5 et présente une viscosité de 19 poises (1,9 Pa·s) mesurée selon le protocole décrit précédemment. Lorsque la composition est conservée à 4°C pendant un mois, elle a un pH de 5,2 et une viscosité de 4,9 poises (0,49 Pa·s) mesurée selon le protocole décrit précédemment. Lorsque la composition est conservée à 45°C pendant un mois, elle a un pH de 5,2 et une viscosité de 5,7 poises (0,57 Pa·s) mesurée selon le protocole décrit précédemment.

Ainsi, ces résultats montrent que seule la composition parfumante selon l'invention, qui contient un copolymère d'acide acrylamido-2-méthyl propane sulfonique ou ses sels et d'un ou plusieurs monomères non ioniques et un copolymère réticulé d'acide acrylique et d'acrylate d'alkyle en C10-C30, est stable et cosmétiquement intéressante.

III/ Une seconde composition selon l'invention selon le Tableau 3 est préparée selon le procédé décrit ci-avant (voir paragraphe I). Cette seconde composition selon l'invention comprend cette fois un homopolymère d'acide acrylamido-2-méthyl propane sulfonique.

**[Tableau 3]**

| **Ingrédients** | **Quantité (% en poids par rapport au poids total de composition) Composition selon l'invention** |
|---|---|
| 2-AMINO-2-METHYL-1-PROPANOLAMINOMETHYL PROPANOL | 0,06 |
| Parfum | 20 |
| Acide poly acrylamidométhyl propane sulfonique neutralisé partiellement à l'ammoniaque et hautement réticulé (Hostacerin AMPS^{®} de chez Clariant) | 0,5 |
| Copolymère réticulé acide acrylique/methacrylate d'alkyl C₁₀-C₃₀ (CARBOPOL ULTREZ 21 POLYMER de chez Lubrizol) | 0,4 |
| GLYCERINE | 2 |
| Ethanol | 21 |
| DIPROPYLENE GLYCOL | 15 |
| Eau | Qsp 100 |

Cette composition selon l'invention a un pH de 5,4 et une viscosité de 52 poises (5,2 Pa·s) mesurée selon le protocole décrit précédemment (avec mobile 4).

La composition présente une bonne stabilité.

## Revendications

1. Composition parfumante, notamment cosmétique et/ou dermatologique, sous forme de gel comprenant:
un milieu aqueux physiologiquement acceptable comprenant au moins de l'éthanol ;
i) un ou plusieurs polymères sulfoniques choisis parmi les homopolymères d'acide acrylamido-2-méthyl propane sulfonique ou ses sels et les copolymères d'acide acrylamido-2-méthyl propane sulfonique ou ses sels et d'un ou plusieurs monomères non ioniques ;
ii) un ou plusieurs copolymères réticulés d'acide acrylique et d'acrylate d'alkyle en C10-C30 ; et
iii) éventuellement un ou plusieurs polymères épaississants organiques différents de i) et ii) ;
au moins 5% en poids par rapport au poids total de composition, d'au moins une substance parfumante.

2. Composition parfumante selon la revendication 1, **caractérisée en ce que** le milieu aqueux comprend au moins un autre solvant organique soluble dans l'eau, à 25°C, choisi parmi les alcanols, linéaires ou ramifiés, en C3-C4, tels que l'isopropanol, le propanol, le butanol; les polyols ayant de 2 à 20 atomes de carbone, de préférence de 2 à 6 atomes de carbone, comme le glycérol, le diglycérol, le propylèneglycol, l'isoprène glycol, le dipropylèneglycol, le butylène glycol, l'hexylène glycol, le 1,3-propanediol, le pentylène glycol, les polyéthylèneglycols ayant de 2 à 200 motifs d'oxyde d'éthylène ; et leurs mélanges.

3. Composition parfumante selon la revendication 1 ou 2, **caractérisée en ce qu'**elle comprend de 15 à 40% en poids d'éthanol par rapport au poids total de la composition, de préférence de 15 à 30% en poids, de préférence de 15 à 25% en poids.

4. Composition parfumante selon l'une des revendications 1 à 3, **caractérisée en ce qu'**elle comprend i) un ou plusieurs homopolymères d'acide acrylamido-2-méthyl propane sulfonique ou ses sels, ledit ou lesdits homopolymères comprenant, distribués de façon aléatoire :
de 90 à 99,9% en poids de motifs de formule générale (1) suivante : 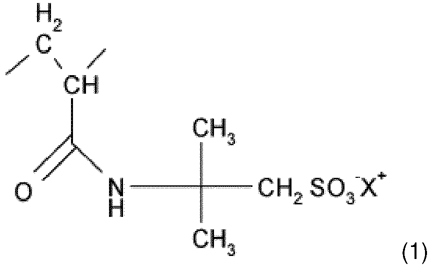
dans laquelle X⁺ désigne un proton, un cation de métal alcalin tel que sodium ou potassium, un cation alcalino-terreux tel que calcium ou l'ion ammonium, au plus 10 % mol des cations X⁺ pouvant être des protons H⁺;
et de 0,01 à 10% en poids de motifs réticulants provenant d'au moins un monomère ayant au moins deux double-liaison oléfiniques,
les proportions en poids étant définies par rapport au poids total du polymère.

5. Composition parfumante selon l'une des revendications 1 à 4, **caractérisée en ce qu'**elle comprend i) un ou plusieurs copolymères d'acide acrylamido-2-méthyl propane sulfonique ou ses sels et d'un ou plusieurs monomères non ioniques, qui sont choisis parmi :
les copolymères d'acide acrylamido-2-methyl propane sulfonique et de vinylpyrrolidone,
les copolymères réticulés acrylamide/acrylamido-2-methyl propane sulfonate de sodium, et
les copolymères d'acide acrylamido-2-methyl propane sulfonique et d'hydroxyéthyl acrylate.

6. Composition parfumante selon l'une des revendications 1 à 5, **caractérisée en ce que** la concentration en homopolymère ou en copolymère d'acide acrylamido-2-methyl propane sulfonique en matière active va de 0,05 à 1% en poids par rapport au poids total de la composition, et de préférence de 0,05 à 0,8% en poids, et encore plus particulièrement de 0,1 à 0,5% en poids par rapport au poids total de la composition.

7. Composition parfumante selon l'une des revendications 1 à 6, **caractérisée en ce que** la concentration en copolymère réticulé d'acide acrylique et d'acrylate d'alkyle en C10-C30 ii) en matière active va de 0,05 à 1% en poids par rapport au poids total de la composition, et de préférence de 0,1 à 0,8% en poids, et encore plus particulièrement de 0,2 à 0,6% en poids par rapport au poids total de la composition.

8. Composition parfumante selon l'une des revendications 1 à 7, **caractérisée en ce qu'**elle comprend de 5% à 30% en poids de substance parfumante, mieux de 10% à 25% en poids, en particulier de 15% à 25% en poids par rapport au poids total de la composition.

9. Composition parfumante selon l'une des revendications 1 à 8, **caractérisée en ce qu'**elle présente une viscosité comprise entre 0,9 à 4 *Pa·s* (9 et 40 Poises), de préférence entre 2,6 à 3,5 *Pa·s* (26 et 35 Poises), de préférence entre 2,6 à 3 *Pa·s* (26 et 30 Poises).

10. Composition parfumante selon l'une des revendications 1 à 9, **caractérisée en ce qu'**elle comprend moins de 2 % en poids par rapport au poids total de la composition d'huile.

11. Composition parfumante selon l'une des revendications 1 à 10, **caractérisée en ce qu'**elle comprend un ou plusieurs polymères épaississants organiques différents de i) et ii), associatifs ou non associatifs, en particulier choisis parmi A) ceux à motifs sucres issus des sucres suivants : glucose ; galactose ; arabinose ; rhamnose ; mannose ; xylose ; fucose ; anhydrogalactose ; acide galacturonique ; acide glucuronique ; acide mannuronique ; galactose sulfate ; anhydrogalactose sulfate et le fructose, telle que a) la gomme arabique (polymère ramifié de galactose, d'arabinose, de rhamnose et d'acide glucuronique) ; b) les carraghénanes et les furcelleranes (polymères de galactose sulfate et d'anhydrogalactose sulfate), c) la gomme de guar (polymère de mannose et de galactose) ; d) la gomme de xanthane (polymère de glucose, de mannose acétate, de mannose/acide pyruvique et d'acide glucuronique) ; e) la gomme de gellane (polymère de glucose partiellement acylé, de rhamnose et d'acide glucuronique) ; f) la gomme de scléroglucane (polymère du glucose) ; g) la cellulose (polymère du glucose) ; h) l'amidon (polymère du glucose) ; i) les éthers de cellulose non ioniques sans chaine grasse en C₁₀-C₃₀ ; B) les polymères issus de la (co)polymérisation de monomère acrylate CH₂=C(R')-COOR"' (VIa) et/ou de monomère acrylamide CH₂=C(R')-CO-N(R")-L-Y⁻ M⁺ (VIb) Formules (Via) et (VIb) dans lesquelles R', et R", identiques ou différents, représentant un atome d'hydrogène ou un groupe (C₁-C₆)alkyle tel que méthyle, de préférence hydrogène, R"' représente un métal alcalin, un métal alcalino-terreux, un atome d'hydrogène ou un groupe (C₁-C₆)alkyle éventuellement substitué notamment par un ou plusieurs groupes hydroxy, carboxy ou amino, de préférence R"' représente un atome d'hydrogène, L représentant un groupe hydrocarboné divalent, cyclique ou acylique, saturé ou insaturé, linéaire ou ramifié, éventuellement interrompu par un ou plusieurs hétéroatomes tels que O, N et comprenant de 1 à 20 atomes de carbones, de préférence de 1 à 6 atomes de carbones, de préférence L représente le groupe divalent -[C(R')(R")]ₚ- avec p représentant un entier compris entre 1 et 4, de préférence 2 et 3 tel que 2, R' et R" étant tels que définis précédemment, plus particulièrement L représente -C(R')(R")-CH₂- ou -CH₂-C(R')(R")- avec R' et R" tels que définis précédemment, de préférence R' et R" représentent un groupe (C₁-C₄)alkyle tel que méthyle ; Y⁻ représente un groupe anionique tel que carboxylate; phosphate, phosphonate, et M⁺ étant un contre ion cationique de préférence métal alcalin tel que sodium; C) les homopolymères d'acrylate d'ammonium ou les copolymères d'acrylate d'ammonium et d'acrylamide ; D) les « *polymères associatifs* » notamment les copolymères comportant parmi leurs monomères un acide carboxylique à insaturation α,β-monoéthylénique et un ester d'acide carboxylique à insaturation α,β-monoéthylénique et d'un alcool gras oxyalkyléné ; m) les (poly)hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci ; les radicaux alkyle portés par les celluloses ou hydroxyéthylcelluloses quaternisées ci-dessus comportent de préférence de 8 à 30 atomes de carbone ; E) les celluloses ou leurs dérivés, modifiés par des groupements comportant au moins une chaîne grasse tels que des groupes alkyls, arylalkyls, alkylaryls ou leurs mélanges où les groupes alkyls sont en C₈- et en particulier les alkylhydroxyéthylcelluloses non-ioniques ; et F) les dérivés de guar associatifs comme les hydroxypropylguars modifiés par une chaîne grasse.

12. Composition parfumante selon l'une des revendications 1 à 11, **caractérisée en ce qu'**elle comprend un ou plusieurs polymères épaississants dans une quantité allant de 0,01 à 10 % en poids plus préférentiellement de 0,1 à 5 % en poids et encore plus préférentiellement 0,01 et 2,5 % en poids par rapport au poids total de la composition.

13. Composition parfumante selon l'une des revendications 1 à 12, **caractérisée en ce qu'**elle consiste un ou plusieurs tensioactifs, en particulier anioniques, amphotères, ou non ioniques, de préférence non ioniques ou anioniques, plus préférentiellement non ioniques.

14. Composition parfumante selon l'une des revendications 1 à 13, **caractérisée en ce qu'**elle consiste en:
un milieu aqueux physiologiquement acceptable comprenant au moins de l'éthanol ;
i) un ou plusieurs polymères sulfoniques choisis parmi les homopolymères d'acide acrylamido-2-méthyl propane sulfonique ou ses sels et les copolymères d'acide acrylamido-2-méthyl propane sulfonique ou ses sels et d'un ou plusieurs monomères non ioniques ;
ii) un ou plusieurs copolymères réticulés d'acide acrylique et d'acrylate d'alkyle en C10-C30 ; et
iii) éventuellement un ou plusieurs polymères épaississants organiques différents de i) et ii) ; et
au moins 5% en poids par rapport au poids total de composition, d'au moins une substance parfumante.

15. Méthode de soin cosmétique et/ou esthétique, de préférence pour parfumer, comprenant l'application topique sur les matières kératiniques d'une composition telle que définie à l'une quelconque des revendications 1 à 14.

## Patentansprüche

1. Riechstoffzusammensetzung, insbesondere kosmetische und/oder dermatologische, in Gelform, umfassend:
ein physiologisch akzeptables wässriges Medium, das mindestens Ethanol umfasst;
i) ein oder mehrere Sulfonpolymere, ausgewählt aus Homopolymeren der Acrylamido-2-methylpropansulfonsäure oder deren Salzen und Copolymeren der Acrylamido-2-methylpropansulfonsäure oder deren Salzen und einem oder mehreren nichtionischen Monomeren;
ii) ein oder mehrere vernetzte Copolymere der Acrylsäure und C10-C30-Alkylacrylat; und
iii) gegebenenfalls ein oder mehrere organische Verdickungspolymere, die von i) und ii) verschieden sind;
mindestens 5 Gew.-%, bezogen auf das Gesamtgewicht der Zusammensetzung, mindestens eines Riechstoffs.

2. Riechstoffzusammensetzung nach Anspruch 1, **dadurch gekennzeichnet, dass** das wässrige Medium mindestens ein weiteres organisches Lösungsmittel umfasst, das bei 25 °C wasserlöslich ist, ausgewählt aus den linearen oder verzweigten C3-C4-Alkanolen wie Isopropanol, Propanol, Butanol; den Polyolen mit 2 bis 20 Kohlenstoffatomen, vorzugsweise 2 bis 6 Kohlenstoffatomen, wie Glycerin, Diglycerin, Propylenglykol, Isoprenglykol, Dipropylenglykol, Butylenglykol, Hexylenglykol, 1,3-Propandiol, Pentylenglykol, den Polyethylenglykolen mit 2 bis 200 Ethylenoxideinheiten; und deren Mischungen.

3. Riechstoffzusammensetzung nach Anspruch 1 oder 2, **dadurch gekennzeichnet, dass** sie 15 bis 40 Gew.-% Ethanol, bezogen auf das Gesamtgewicht der Zusammensetzung, vorzugsweise 15 bis 30 Gew.-%, vorzugsweise 15 bis 25 Gew.-%, umfasst.

4. Riechstoffzusammensetzung nach einem der Ansprüche 1 bis 3, **dadurch gekennzeichnet, dass** sie i) ein oder mehrere Homopolymere der Acrylamido-2-methylpropansulfonsäure oder deren Salze umfasst, wobei das oder die Homopolymere in zufälliger Verteilung umfassen:
90 bis 99,9 Gew.-% Einheiten der folgenden allgemeinen Formel (1): 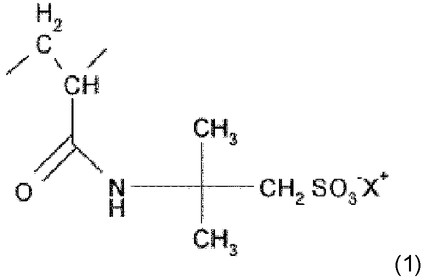
wobei X⁺ ein Proton, ein Alkalimetallkation wie Natrium oder Kalium, ein Erdalkalimetallkation wie Calcium oder das Ammoniumion bezeichnet, wobei höchstens 10 Mol-% der X⁺-Kationen H⁺-Protonen sein können;
und 0,01 bis 10 Gew.-% an vernetzenden Einheiten, die von mindestens einem Monomer mit mindestens zwei olefinischen Doppelbindungen stammen,
wobei die Gewichtsanteile in Bezug auf das Gesamtgewicht des Polymers definiert sind.

5. Riechstoffzusammensetzung nach einem der Ansprüche 1 bis 4, **dadurch gekennzeichnet, dass** sie i) ein oder mehrere Copolymere der Acrylamido-2-methylpropansulfonsäure oder deren Salze und ein oder mehrere nichtionische Monomere umfasst, die ausgewählt sind aus:
Copolymeren der Acrylamido-2-methylpropansulfonsäure und Vinylpyrrolidon, vernetzten Copolymeren von Acrylamid/Natriumacrylamido-2-methylpropansulfonat und
Copolymeren der Acrylamido-2-methylpropansulfonsäure und Hydroxyethylacrylat.

6. Riechstoffzusammensetzung nach einem der Ansprüche 1 bis 5, **dadurch gekennzeichnet, dass** die Konzentration an Homopolymer oder Copolymer der Acrylamido-2-methylpropansulfonsäure als Wirkstoff 0,05 bis 1 Gew.-%, bezogen auf das Gesamtgewicht der Zusammensetzung, und vorzugsweise 0,05 bis 0,8 Gew.-%, und insbesondere 0,1 bis 0,5 Gew.-%, bezogen auf das Gesamtgewicht der Zusammensetzung, beträgt.

7. Riechstoffzusammensetzung nach einem der Ansprüche 1 bis 6, **dadurch gekennzeichnet, dass** die Konzentration an vernetztem Copolymer der Acrylsäure und C10-C30-Alkylacrylat ii) als Wirkstoff 0,05 bis 1 Gew.-%, bezogen auf das Gesamtgewicht der Zusammensetzung, und vorzugsweise 0,1 bis 0,8 Gew.-%, und insbesondere 0,2 bis 0,6 Gew.-%, bezogen auf das Gesamtgewicht der Zusammensetzung, beträgt.

8. Riechstoffzusammensetzung nach einem der Ansprüche 1 bis 7, **dadurch gekennzeichnet, dass** sie 5 bis 30 Gew.-% Riechstoff, besser 10 bis 25 Gew.-%, vor allem 15 bis 25 Gew.-%, bezogen auf das Gesamtgewicht der Zusammensetzung, umfasst.

9. Riechstoffzusammensetzung nach einem der Ansprüche 1 bis 8, **dadurch gekennzeichnet, dass** sie eine Viskosität zwischen 0,9 und 4 *Pa·s* (9 und 40 Poises), vorzugsweise zwischen 2,6 und 3,5 *Pa·s* (26 und 35 Poises), vorzugsweise zwischen 2,6 und 3 *Pa·s* (26 und 30 Poises) aufweist.

10. Riechstoffzusammensetzung nach einem der Ansprüche 1 bis 9, **dadurch gekennzeichnet, dass** sie weniger als 2 Gew.-%, bezogen auf das Gesamtgewicht der Zusammensetzung, Öl umfasst.

11. Riechstoffzusammensetzung nach einem der Ansprüche 1 bis 10, **dadurch gekennzeichnet, dass** sie ein oder mehrere von i) und ii) verschiedene, assoziative oder nicht assoziative organische Verdickungspolymere umfasst, die vor allem ausgewählt sind aus A) solchen mit Zuckereinheiten, die von den folgenden Zuckern abgeleitet sind: Glucose; Galactose; Arabinose; Rhamnose; Mannose; Xylose; Fucose; Anhydrogalactose; Galacturonsäure; Glucuronsäure; Mannuronsäure; Galactosesulfat; Anhydrogalactosesulfat und Fructose, wie a) Gummi arabicum (verzweigtes Polymer von Galactose, Arabinose, Rhamnose und Glucuronsäure); b) den Carrageenanen und den Furcelleranen (Polymere von Galactosesulfat und Anhydrogalactosesulfat), c) Guargummi (Polymer von Mannose und Galactose); d) Xanthangummi (Polymer von Glucose, Mannoseacetat, Mannose/Pyruvinsäure und Glucuronsäure); e) Gellangummi (Polymer von teilweise acylierter Glucose, Rhamnose und Glucuronsäure); f) Skleroglucangummi (Glucosepolymer); g) Cellulose (Glucosepolymer); h) Stärke (Glucosepolymer); i) nichtionische Celluloseether ohne C₁₀-C₃₀-Fettkette; B) den Polymeren, die aus der (Co)polymerisation von Acrylatmonomer CH₂=C(R')-COOR'" (VIa) und/oder Acrylamidmonomer CH₂=C(R')-CO-N(R")-L-Y-M⁺ (VIb) Formeln (VIa) und (Vlb) abgeleitet sind, worin R', und R", die gleich oder verschieden sind, ein Wasserstoffatom oder eine (C₁-C₆)-Alkylgruppe wie Methyl, vorzugsweise Wasserstoff, darstellen, R‴ ein Alkalimetall, ein Erdalkalimetall, ein Wasserstoffatom oder eine (C₁-C₆)-Alkylgruppe darstellt, gegebenenfalls insbesondere durch eine oder mehrere Hydroxy-, Carboxy- oder Aminogruppen substituiert, vorzugsweise R‴ ein Wasserstoffatom darstellt, wobei L eine zweiwertige, cyclische oder acylische, gesättigte oder ungesättigte, lineare oder verzweigte Kohlenwasserstoffgruppe darstellt, gegebenenfalls unterbrochen durch ein oder mehrere Heteroatome wie O, N und 1 bis 20 Kohlenstoffatome, vorzugsweise 1 bis 6 Kohlenstoffatome, umfasst, wobei L vorzugsweise die zweiwertige Gruppe -[C(R')(R")]ₚ- darstellt mit p, das eine ganze Zahl zwischen 1 und 4, vorzugsweise 2 und 3, wie 2 darstellt, wobei R' und R" wie vorstehend definiert sind, insbesondere L -C(R')(R")-CH₂ oder -CH₂-C(R')(R")- darstellt mit R' und R" wie vorstehend definiert, wobei vorzugsweise R' und R" eine (C₁-C₄)-Alkylgruppe wie Methyl darstellen, Y eine anionische Gruppe wie Carboxylat; Phosphat, Phosphonat darstellt, und M⁺ ein kationisches Gegenion, vorzugsweise Alkalimetall wie Natrium ist; C) den Ammoniumacrylat-Homopolymeren oder Ammoniumacrylat- und Acrylamid-Copolymeren; D) den "*assoziativen Polymeren*", insbesondere den Copolymeren, die unter ihren Monomeren eine α,β-monoethylenisch ungesättigte Carbonsäure und einen α,β-monoethylenisch ungesättigten Carbonsäure- und alkylenoxidierten Fettalkoholester enthalten; m) den quaternisierten (Poly)hydroxyethylcellulosen, die mit Gruppen modifiziert sind, die mindestens eine Fettkette enthalten, wie Alkyl-, Arylalkyl-, Alkylarylgruppen mit mindestens 8 Kohlenstoffatomen, oder deren Mischungen; die Alkylreste, die von den obigen Cellulosen oder quaternisierten Hydroxyethylcellulosen getragen werden, weisen vorzugsweise 8 bis 30 Kohlenstoffatome auf; E) den Cellulosen oder deren Derivaten, die mit Gruppen modifiziert sind, die mindestens eine Fettkette wie Alkyl-, Arylalkyl-, Alkylarylgruppen oder deren Mischungen enthalten, wobei die Alkylgruppen C₈₋ sind und insbesondere die nichtionischen Alkylhydroxyethylcellulosen; und F) assoziative Guarderivate wie mit einer Fettkette modifizierte Hydroxypropylguare.

12. Riechstoffzusammensetzung nach einem der Ansprüche 1 bis 11, **dadurch gekennzeichnet, dass** sie ein oder mehrere Verdickungspolymere in einer Menge von 0,01 bis 10 Gew.-%, bevorzugt von 0,1 bis 5 Gew.-% und noch bevorzugt von 0,01 und 2,5 Gew.-%, bezogen auf das Gesamtgewicht der Zusammensetzung, umfasst.

13. Riechstoffzusammensetzung nach einem der Ansprüche 1 bis 12, **dadurch gekennzeichnet, dass** sie aus einem oder mehreren Tensiden, insbesondere anionischen, amphoteren oder nichtionischen, vorzugsweise nichtionischen oder anionischen, bevorzugter nichtionischen Tensiden, besteht.

14. Riechstoffzusammensetzung nach einem der Ansprüche 1 bis 13, **dadurch gekennzeichnet, dass** sie besteht aus:
ein physiologisch akzeptables wässriges Medium, das mindestens Ethanol umfasst;
i) ein oder mehrere Sulfonpolymere, ausgewählt aus Homopolymeren der Acrylamido-2-methylpropansulfonsäure oder deren Salzen und Copolymeren der Acrylamido-2-methylpropansulfonsäure oder deren Salzen und einem oder mehreren nichtionischen Monomeren;
ii) ein oder mehrere vernetzte Copolymere der Acrylsäure und C10-C30-Alkylacrylat; und
iii) gegebenenfalls einem oder mehreren organischen Verdickungspolymeren, die sich von i) und ii) unterscheiden; und
mindestens 5 Gew.-%, bezogen auf das Gesamtgewicht der Zusammensetzung, mindestens eines Riechstoffs.

15. Verfahren zur kosmetischen und/oder ästhetischen Pflege, vorzugsweise zum Parfümieren, umfassend das topische Auftragen einer Zusammensetzung nach einem der Ansprüche 1 bis 14 auf Keratinmaterialien.

## Claims

1. A perfuming composition, in particular cosmetic and/or dermatological, in the form of a gel comprising:
a physiologically acceptable aqueous medium comprising at least ethanol;
i) one or more sulphonic polymers chosen from homopolymers of 2-acrylamido-2-methylpropanesulphonic acid or its salts and copolymers of 2-acrylamido-2-methylpropanesulphonic acid or its salts and one or more nonionic monomers;
ii) one or more cross-linked copolymers of acrylic acid and C10-C30 alkyl acrylate; and
iii) optionally one or more organic thickening polymers different from i) and ii);
at least 5% by weight, relative to the total weight of the composition, of at least one perfuming substance.

2. The perfuming composition according to claim 1, **characterised in that** the aqueous medium comprises at least one other organic solvent that is soluble in water at 25°C, chosen from linear or branched C3-C4 alkanols, such as isopropanol, propanol, butanol, polyols having from 2 to 20 carbon atoms, preferably from 2 to 6 carbon atoms, such as glycerol, diglycerol, propylene glycol, isoprene glycol, dipropylene glycol, butylene glycol, hexylene glycol, 1,3-propanediol, pentylene glycol, polyethylene glycols having from 2 to 200 ethylene oxide units; and mixtures thereof.

3. The perfuming composition according to claim 1 or 2, **characterised in that** it comprises from 15 to 40% by weight of ethanol relative to the total weight of the composition, preferably from 15 to 30% by weight, more preferably from 15 to 25% by weight.

4. The perfuming composition according to one of claims 1 to 3, **characterised in that** it comprises i) one or more homopolymers of 2-acrylamido-2-methylpropanesulphonic acid or its salts, said homopolymer(s) comprising, distributed randomly:
from 90 to 99.9% by weight of units of the following general formula (1): 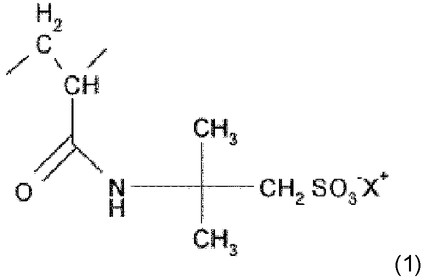
wherein X⁺ denotes a proton, an alkali metal cation such as sodium or potassium, an alkaline earth cation such as calcium or the ammonium ion, it being possible for at most 10 mol% of the cations X⁺ to be H⁺ protons;
and from 0.01 to 10% by weight of crosslinking units derived from at least one monomer having at least two olefinic double bonds,
the proportions by weight being defined in relation to the total weight of the polymer.

5. The perfuming composition according to one of claims 1 to 4, **characterised in that** it comprises i) one or more copolymers of 2-acrylamido-2-methylpropanesulphonic acid or its salts and of one or more nonionic monomers, which are chosen from:
copolymers of 2-acrylamido-2-methylpropanesulphonic acid and vinylpyrrolidone, cross-linked acrylamide/2-acrylamido-2-methylpropanesulphonic acid sodium salt copolymers, and
copolymers of 2-acrylamido-2-methylpropanesulphonic acid and hydroxyethyl acrylate.

6. The perfuming composition according to one of claims 1 to 5, **characterised in that** the concentration of homopolymer or copolymer of 2-acrylamido-2-methylpropanesulphonic acid as active material ranges from 0.05 to 1% by weight relative to the total weight of the composition, and preferably from 0.05 to 0.8% by weight, and even more particularly from 0.1 to 0.5% by weight relative to the total weight of the composition.

7. The perfuming composition according to one of claims 1 to 6, **characterised in that** the concentration of crosslinked copolymer of acrylic acid and C10-C30 alkyl acrylate ii) as active material ranges from 0.05 to 1% by weight relative to the total weight of the composition, and preferably from 0.1 to 0.8% by weight, and even more particularly from 0.2 to 0.6% by weight relative to the total weight of the composition.

8. The perfuming composition according to one of claims 1 to 7, **characterised in that** it comprises from 5% to 30% by weight of perfuming substance, better still from 10% to 25% by weight, in particular from 15% to 25% by weight relative to the total weight of the composition.

9. The perfuming composition according to one of claims 1 to 8, **characterised in that** it has a viscosity of between 0.9 and 4 *Pa·s* (9 and 40 Poises), preferably between 2.6 and 3.5 *Pa·s* (26 and 35 Poises), more preferably between 2.6 and 3 *Pa·s* (26 and 30 Poises).

10. The perfuming composition according to one of claims 1 to 9, **characterised in that** it comprises less than 2% by weight relative to the total weight of the oil composition.

11. The perfuming composition according to one of claims 1 to 10, **characterised in that** it comprises one or more associative or non-associative organic thickening polymers other than i) and ii), in particular chosen from A) those containing sugar units derived from the following sugars: glucose; galactose; arabinose; rhamnose; mannose; xylose; fucose; anhydrogalactose; galacturonic acid; glucuronic acid; mannuronic acid; galactose sulphate; anhydrogalactose sulphate and fructose, such as a) gum arabic (branched polymer of galactose, arabinose, rhamnose and glucuronic acid); b) carrageenans and furcellerans (polymers of galactose sulphate and anhydrogalactose sulphate), c) guar gum (polymer of mannose and galactose); d) xanthan gum (polymer of glucose, mannose acetate, mannose/pyruvic acid and glucuronic acid); e) gellan gum (polymer of partially acylated glucose, rhamnose and glucuronic acid); f) scleroglucan gum (glucose polymer); g) cellulose (glucose polymer); h) starch (glucose polymer); i) non-ionic cellulose ethers without a C₁₀-C₃₀ fatty chain; B) polymers derived from the (co)polymerisation of acrylate monomer CH₂=C(R')-COOR'" (Vla) and/or acrylamide monomer CH₂=C(R')-CO-N(R")-L-Y- M⁺ (Vlb) Formulae (Vla) and (Vlb) in which R' and R", which may be identical or different, represent a hydrogen atom or a (C₁-C₆)alkyl group such as methyl, preferably hydrogen, R'" represents an alkali metal, an alkaline-earth metal, a hydrogen atom or a (C₁-C₆)alkyl group optionally substituted in particular by one or more hydroxyl, carboxyl or amino groups, preferably R‴ represents a hydrogen atom, L representing a divalent hydrocarbon group, cyclic or acyclic, saturated or unsaturated, linear or branched, optionally interrupted by one or more heteroatoms such as O, N and comprising from 1 to 20 carbon atoms, preferably from 1 to 6 carbon atoms, preferably L represents the divalent group -[C(R')(R")]ₚ- with p representing an integer between 1 and 4, preferably 2 and 3 such as 2, R' and R" being as defined above, more particularly L represents -C(R')(R")- CH₂- or -CH₂-C(R')(R")- where R' and R" are as defined above, preferably R' and R" represent a (C₁-C₄)alkyl group such as methyl; Y represents an anionic group such as carboxylate; phosphate, phosphonate, and M⁺ a cationic counterion, preferably an alkali metal such as sodium; C) ammonium acrylate homopolymers or ammonium acrylate/acrylamide copolymers; D) "*associative polymers*", in particular copolymers comprising, among their monomers, an α,β-monoethylenically unsaturated carboxylic acid and an ester of an α,β-monoethylenically unsaturated carboxylic acid and of an oxyalkylenated fatty alcohol; m) quaternised (poly(hydroxyethylcellulose)s modified with groups containing at least one fatty chain, such as alkyl, arylalkyl or alkylaryl groups containing at least 8 carbon atoms, or mixtures thereof; the alkyl radicals carried by the above quaternised celluloses or hydroxyethylcelluloses preferably contain from 8 to 30 carbon atoms; E) celluloses or their derivatives modified by groups containing at least one fatty chain such as alkyl, arylalkyl, alkylaryl groups or mixtures thereof where the alkyl groups are C₈ - and in particular non-ionic alkylhydroxyethylcelluloses; and F) associative guar derivatives such as hydroxypropylguars modified by a fatty chain.

12. The perfuming composition according to one of claims 1 to 11, **characterised in that** it comprises one or more thickening polymers in an amount ranging from 0.01 to 10% by weight, more preferably from 0.1 to 5% by weight and even more preferably from 0.01 to 2.5% by weight relative to the total weight of the composition.

13. The perfuming composition according to one of claims 1 to 12, **characterised in that** it consists of one or more surfactants, in particular anionic, amphoteric or nonionic, preferably nonionic or anionic, more preferably nonionic.

14. The perfuming composition according to one of claims 1 to 13, **characterised in that** it consists of:
a physiologically acceptable aqueous medium comprising at least ethanol;
i) one or more sulphonic polymers chosen from homopolymers of 2-acrylamido-2-methylpropanesulphonic acid or its salts and copolymers of 2-acrylamido-2-methylpropanesulphonic acid or its salts and one or more nonionic monomers;
ii) one or more cross-linked copolymers of acrylic acid and C10-C30 alkyl acrylate; and
iii) optionally one or more organic thickening polymers other than i) and ii); and
at least 5% by weight, relative to the total weight of the composition, of at least one perfuming substance.

15. A cosmetic and/or aesthetic care method, preferably for perfuming, comprising the topical application to keratinous materials of a composition as defined in any one of claims 1 to 14.
